# EUROPEAN PATENT APPLICATION

(11) **EP 2 829 600 A1**
(43) Date of publication of application: **28.01.2015**
(21) Application number: 13177369.9
(22) Date of filing: 22.07.2013
(51) Int. Cl.: C12N 5/0784, C12N 5/0783

(54) **Method for preparing dendritic cells to be used as antigen-presenting cells in immunotherapy**

(71) Applicant: Sotio a.s., 170 00 Prague 7 (CZ)
(72) Inventor: Truxova, Iva, 77900 Olomouc (CZ); Fucikova, Jitka, 58601 Jihlava (CZ); Spisek, Radek, Dr., 14800 Prag (CZ); Bartunkova, Jirina, Prof. Dr., 11000 Prag (CZ)
(74) Representative: Keller, Günter

(57) **Abstract**

The present invention discloses an in vitro method for preparing dendritic cells as antigen-presenting cells for use in immunotherapy whereby the method comprises the following steps:
a) isolation of peripheral blood mononuclear cells,
b) isolation of monocytes from peripheral blood mononuclear cells by adherence to plastic material,
c) culturing the monocytes in serum free medium for the cultivation of dendritic cells for up to 80 hours and
d) activation of the cultured monocyte cells with 10-50 pg/ml Poly(I:C) for up to 28 hours.

## Description

Dendritic cells (DCs) are considered essential for the initiation, programming and regulation of antigen-specific immune responses. They play a fundamental role in supporting the survival and the effector function of primed T cells while coordinating communication among cells of the immune system. The unique ability of dendritic cells to prime naive T cells and to elicit and induce effective cytotoxic T lymphocyte responses resulted in the frequent use of DCs in the preparation of vaccines in cancer therapy.

### Summary of the invention

Dendritic cells (DCs) are professional antigen-presenting cells (APCs) that are capable of inducing immune responses. DC-based vaccines are normally generated using a standard 5- to 7-day protocol for culturing blood monocytes in the presence of cytokines IL-4 and GM-CSF. To shorten the vaccine production for use in cancer immunotherapy and to generate DCs with quality GMP in a shorter time, a fast DC protocol has been developed. Standard DCs (5 days-Day 5 DCs) and fast DCs (3 days-Day 3 DCs) were compared. In this application, the generation of Day 5 versus Day 3 DCs was tested using CellGro, a GMP culture media and subsequent activation by two activation stimuli: Poly(I:C) (TLR-3) and LPS (TLR-4). Although LPS is known as highly efficient activation agent it cannot be used in medical preparations. DC morphology, viability, phagocyte activity, cytokine production and ability to stimulate antigen-specific T cells was evaluated. The results demonstrate that, although Day 5 DCs were larger in size compared to Day 3 DCs, they exhibited similar phagocytic capacity. The viability of Day 5 and Day 3 DCs was comparable (>80%). Poly(I:C)-activated Day 5 DCs expressed higher levels of the costimulatory and surface molecules CD80, CD86 and HLA-DR compared to Poly(I:C)-activated Day 3 DCs. Nevertheless, LPS-activated Day 5 and Day 3 DCs were phenotypically similar. Cytokine production was generally stronger when LPS was used as the maturation stimulus, and there were no significant differences between Day 5 and Day 3 DCs. Furthermore, Poly(I:C)-activated Day 5 DCs secreted higher amounts of IL-6 and IFN-α compared to Day 3 DCs. Importantly, Day 5 and Day 3 DCs were able to generate comparable numbers of antigen-specific T cells, and the number of Tregs induced by Day 5 and Day 3 DCs were also comparable. Monocyte-derived DCs generated in CellGro^{®} for 3 days and activated using Poly(I:C) were identified as being similarly potent as clinical-grade DCs, when compared to DCs produced by the standard 5 day protocol. These results provide the rationale for the evaluation of faster protocols for DC generation in clinical trials. Generating fast DCs markedly reduces the time, work load and cost associated with *in vitro* culture of DC precursors and, thus, may facilitate the use of DCs in clinical trials of cellular immunotherapy.

### Prior art

Castiello et al., Cancer Immunol. Immunother. (2011), pp 457-466, describe different monocyte-derived DC maturation strategies. Several strategies are summarized which lead to dendritic cells which can be used for immunotherapy.

Rouas et al., International Immunology (2004), pp 767-773, describe the preparation of dendritic cells whereby peripheral blood mononuclear cells (PBMCs) were cultured for six days in a suitable medium, then further cultured for two days in the presence of GM-CSF and IL-4 and activated with Poly(I:C). Mature dendritic cells were harvested after eight days of culture.

Fu 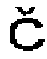 íková et al., Journal of Translational Medicine (2011), 9:223, discloses dendritic cells for use in cancer immunotherapy trials. The use of a maturation cocktail comprising TNF-α, IL-1β, IL-6 and PTE2 resulted in a high population of undesired regulatory T cells (T_{reg}).

It is, however, an object of the present invention to provide dendritic cells which can be used for tumor vaccination after a shorter period of time.

### Detailed description of the invention

Dendritic cells (DCs) are the most potent antigen-presenting cells in the human immune system. Due to their unique ability to prime and stimulate both CD8⁺ and CD4⁺ T cells, DCs loaded with whole tumor cells and tumor lysates have been investigated in a number of clinical trials for their ability to induce anti-tumor T cell responses. Encouraging immunologic and therapeutic responses have been observed in cancer patients treated with DC vaccines, leading to an increased interest in developing protocols for the clinical-scale production of DCs.

Human DCs can be divided into at least three subpopulations: interstitial DCs that are located in the skin and lymphoid organs, and the two subsets of DCs in the blood, which are the CD11c⁺ myeloid and CD11c⁻ plasmacytoid DCs. Nevertheless, circulating blood DCs are rare, and they are difficult to isolate and maintain in culture. The most widely used DCs for clinical trials are the myeloid DCs that are differentiated from peripheral blood monocytes in the presence of recombinant granulocyte-macrophage colony-stimulating factor (GM-CSF) and interleukin 4 (IL-4). In most trials, 5- to 7-day protocols are used to generate fully differentiated DCs. These DCs resemble immature DCs and exhibit a high phagocytic capacity. When they encounter proinflammatory, microbial, or T cell-derived stimuli, Day 7 DCs become activated and upregulate surface markers such as CD80, CD86, CD40, MHC and chemokine receptors, such as CCR7. Therefore, mature DCs acquire the capacity to migrate to the T cell areas of draining secondary lymphoid organs, where they encounter naive T cells and induce antigen-specific T cell responses.

For practical use it is now mandatory that DC-based vaccine manufacturing complies with the strict guidelines of regulatory agencies for use of cellular therapy products and proceeds according to Good Manufacturing Practices (GMPs). This process includes validated clean rooms, trained staff and GMP materials. These requirements make the manufacturing of DC-based products laborious and costly. Therefore, to manufacture DC-based products for cancer immunotherapy, shorter DC differentiation protocols have been investigated to reduce the amount of lab work, increase the turnover rate in clean boxes and increase the capacity of GMP facilities. Several groups have established short-term experimental protocols in which monocytes were exposed for 2-3 days to recombinant GM-CSF and IL-4 (Dauer et al., J.Immunol. (2003), 4069-4076). A shortened version of the standard regulatory agency's certified protocol was evaluated. The generation of Day 5 (standard) DCs versus Day 3 (fast) DCs was compared using GMP compliant reagents, and the morphology, viability and endocytic capacity of the cells was evaluated. The activation status of Day 5 versus Day 3 DCs was investigated after treatment with either Poly(I:C) (TLR-3) or LPS (TLR-4), and their ability to produce cytokines (IL-12p70, IL-6, IL-10, IL-1β, TNF-α and IFN-α). Additionally, the capacity of activated Day 5 and Day 3 DCs to induce antigen-specific IFN-γ T cell responses was tested. It could be demonstrated that Day 3 clinical grade monocyte-derived DCs are comparable to standard DCs in all important functional aspects. Thus, the method disclosed herein provides important preclinical data for more flexible and cost effective modifications of GMP compliant protocols for clinical grade DC generation.

The present invention provides an in vitro method for preparing dendritic cells which can be used as antigen-presenting cells in immunotherapy, in particular in the vaccination against tumors. Preferably the dendritic cells are used for vaccination against prostatic cancer and/or ovarian cancer and/or lung cancer.

In the first step of the method peripheral blood mononuclear cells (PBMCs) are isolated. In a preferred embodiment the PBMCs are isolated from the buffy coat fraction of centrifuged blood samples obtained from the donor. In a particularly preferred embodiment the donor is the patient to be treated with the vaccinating agent. Although the PBMCs are preferably obtained from the patient who will be treated with the antitumor vaccination it is also possible to obtain the PBMCs from other sources, e.g. from healthy volunteers. For obtaining a buffy coat fraction anticoagulated blood sample is centrifuged. Between the red blood cells on the bottom of the centrifugation tube and the plasma layer on top there is a layer designated as buffy coat which comprises mainly leukocytes and platelets.

The buffy coat layer is isolated and the monocytes contained within the PBMCs are enriched by adherence to plastic material. This can be obtained by transferring the suspended buffy coat or the PBMCs obtained by another method to a plastic material, preferably a cell growth bottle made from plastic material, preferably polystyrene. The cells which do not adhere to plastic material can be removed preferably by rinsing the bottle with CellGro medium.

According to the teaching of the present invention the monocytes are separated from the other peripheral blood moncytic cells (PBMC) by adherence to plastic. Although it seems that this method of separation does not result in highly purified populations of monocytes. The populations produced according to the teaching of the present invention can contain up to 30%, preferably up to 20% of lymphocytes. The isolation of monocytes by the use of magnetic beads and suitable specific linkers results in purer population. The separation by adherence to plastic material, however, seems - possibly due to the fact of the presence of lymphocytes - to result in more potent dendritic cells after maturation, It is also possible that the isolation of CD14⁺ monocytes can induce signaling cascades through the binding of the anti-CD14 antibody. This may be another possible explanation for obtaining less potent dendritic cells. Consequently, although the separation by adherence results in a less pure population, nevertheless the immature dendritic cells obtained by the method disclosed herein seem to be more efficient in immunotherapy.

Then the monocytes are cultured in the cell free medium for a time period up to 80 hours, preferably 65 to 80 hours, more preferred 70 to 74 hours. In a preferred embodiment of the present invention the cell free medium is commercially available from CellGenix, Freiburg. Said medium is sold under the trademark CELLGRO^{®} in Europe. It contains usual components of a cell growth medium, namely salts, sugars, amino acids, vitamins, buffers and as pH indicator phenol-red. Furthermore, it contains L-glutamine and transferrin (human plasma-derived), albumin (human plasma-derived) and insulin which is preferably recombinantly produced. The medium has an osmolality of 260-320 mOsm/kg H₂O and a pH value of about 7.2-7.5. During this cultivation dendritic cells are generated from the monocytes.

The media used in the present invention do not contain serum. In the prior art frequently a medium having the designation "RPMI1640" which is supplemented with 2% human serum is used. The disadvantage thereof is, however, that even minor variations of the batches may occur due to different batches of serum. Furthermore, products derived from human serum do potentially comprise the risk of infection with an infectious agent, like not fully inactivated nucleic acids derived from viruses. The present invention provides therefore a more reproducible and safer result.

In a further preferred embodiment the cultivation of the monocytes is performed in presence of granulocyte macrophage colony-stimulating factor (GM-CSF) which is preferably recombinantly produced. The concentration of GM-CSF ranges from 350 to 700 units/ml medium, preferably from 450 to 550 units/ml. Furthermore, the serum free medium contains 10-50 ng/ml of IL-4, preferably 20-35 ng/ml of IL-4. Interleukin-4 (IL-4) is a cytokin that induces differentiation of naive helper T cells to Th2 cells.

After a cultivation time ranging from 65 to 80 hours in serum free CellGro medium the cultured monocytes are activated by adding 10-50 µg/ml Poly(I:C). polyinosinic: polycitidylic acid [Poly(I:C)] is generally known as an immunostimulant. Poly(I:C) is structurally similar to double-stranded RNA which occurs in some viruses. It is used as a natural stimulant of the toll-like receptor (TLR3). The activation of the cultured monocytes / dendritic cells is performed by cultivation with Poly(I:C) for a period up to 28 hours, preferably from 4 to 28 hours, more preferred from 12 to 24 hours.

The dendritic cells obtained by the method described herein are preferably used for the preparation of a vaccine against tumors. The dendritic cells are brought into contact with specifically treated tumor cells. It is preferred to use autologous tumor cells from the patient to be treated. Said tumor cells are preferably transferred to an apoptotic stage and combined with the dendritic cells. Those cells are then used for vaccination.

The production of DCs for clinical use has to comply with the European Medicines Agency guideline on advanced therapy medicinal products EC1394/2007. These guidelines include requirements of validated clean rooms, trained staff and GMP certified materials, which makes the manufacturing of DC-based products laborious, time consuming and costly. Therefore, modifications of standard protocols for DC generation are sought to reduce the amount of lab work, increase the turnover rate in clean boxes and increase the capacity of GMP facilities while still maintaining the functional characteristics of generated DCs. DCs are typically differentiated from blood monocytes for 5-7 days in the presence of GM-CSF and IL-4 and then activated by proinflammatory or microbial stimuli for another 1-3 days (standard DCs). The shorter version of the protocol for clinical grade DC differentiation was used for the production of DCs for cancer immunotherapy clinical trials. Generated DCs were tested for several criteria that reflect their function, such as upregulation of maturation markers, expression of costimulatory molecules, cytokine production, capacity to activate antigen-specific T cells and capacity to expand regulatory T cells; and these criteria were compared to standard Day 5 DCs.

There have been many studies carried out for example by Dauer et al., J.Immunology, 2003, pp 4069-4076, regarding rapid generation of DCs. To test a shorter protocol for DC generation that is compatible with clinical use, the monocyte-derived DCs in GMP-certified CellGro DC media were cultured, and DCs were activated using Poly(I:C) that is available in GMP quality. Such DCs can be differentiated from human monocytes within 3 days of *in vitro* culture. Three day- and 5 day-cultivation of adherent monocytes in CellGro media in the presence of GM-CSF and IL-4 yielded similar numbers of viable immature DCs. Although FSC/SSC analysis showed that Day 5 DCs were larger in size with higher granularity, Day 3 DCs had the same phagocytic capacity as Day 5 DCs.

In the majority of their experiments the authors of the publication Dauer et al. compare their fast dendritic cells (only two day culture technique) to dendritic cells cultivated for two days in the presence of GM-CSF and IL-4 and not to a cultivation of 5-7 days in GM-CSF and IL-4. Therefore, it is difficult to evaluate these experiments in comparison to the technique described in the present application.

The maturation status of DC is crucial for adequate T cell recruitment, activation, expansion and differentiation. Therefore, the capacity of Poly(I:C) and LPS to activate Day 5 and Day 3 DCs was tested. The maturation of DCs with LPS resulted in the highest expression of maturation-associated molecules with no significant differences between Day 5 and Day 3 DCs. Poly(I:C) induced weaker phenotypic maturation than LPS, and Day 5 Poly(I:C)-activated DCs expressed higher levels of CD80, CD86 and HLA-DR compared to Day 3 DCs.

Next the production of proinflammatory cytokines IL-12p70, IL-6, IL-1β, TNF-α, IFN-α, and anti-inflammatory cytokine IL-10, by activated DCs was analyzed. LPS-induced production of all cytokines tested, except IFN-α, lacked a significant difference between Day 5 and Day 3 DCs. In contrast, Poly(I:C) was only able to induce the production of IL-6 and IFN-α. DCs activated by Poly(I:C) did not produce IL-10, an immunosuppressive cytokine, which is known to induce tolerance by directly inhibiting the production of IL-12 and thereby Th1 cell polarization. IL-12 is a key cytokine for the generation of Th1 cells, CTL and anti-tumor responses. Moreover, IL-12 production together with the mature state of DCs appears to correlate with therapeutic efficacy in clinical trials. Therefore, for DC-based immunotherapy, in cancer as well as in chronic infectious diseases, it is crucial to generate stable mature DCs that secrete bioactive IL-12.

The fact that Poly(I:C) is not able to stimulate DC maturation to the same level as LPS and that Poly(I:C)-activated DCs do not produce any IL-12 has been described.

However, for DC-based immunotherapy, a major concern is whether mature DCs are capable of secreting IL-12p70 after *in vivo* administration. Rouas et al. (Int.Immunol., 2004, 767-773) showed that *in vitro* generated Poly(I:C)-activated DCs retain their capacity to produce IL-12 after migration to the lymph nodes and upon subsequent contact with activated T cells. It has been shown that DC interaction to T cells via CD40:CD40L upregulates the expression of costimulatory and adhesion molecules on DCs and triggers DCs to secrete IL-12. These data suggest that ligation of CD40 on DCs may be an additional way to boost IL-12 production.

The ability to activate antigen-specific T cells is critical for the clinical effect of DC-based vaccines. Therefore, an HLA-A2-restricted influenza MP and CEF peptide mix was used as model antigens to assess the activating potential of the generated Day 5 and Day 3 DCs. Interestingly, DCs that were activated by Poly(I:C) were slightly more efficient at inducing antigen-specific T cells compared to LPS-activated DCs, especially when using CEF peptide mix-pulsed DCs. Antigen-loaded Poly(I:C), as well as LPS-activated, Day 3 DCs acquired full T cell stimulatory capacity and, thus, were equally effective at inducing the expansion of antigen-specific T cells compared to Day 5 DCs. Furthermore, Poly(I:C)- and LPS-activated Day 3 DCs induced slightly lower numbers of regulatory T cells compared to Day 5 DCs.

The method described herein provides monocyte-derived clinical grade DCs generated in accordance with the GMP standards during a 3 day culture process, and these cells, when activated by Poly(I:C), were comparable in all aspects to DCs produced by the standard 5 day protocol. These results provide the rationale for the testing of shorter protocols for DC generation in clinical trials. The generation of DCs using shorter protocols would markedly reduce the time, work load and costs associated with the GMP-compliant manufacturing of DC-based cellular therapy products, and may therefore facilitate the use of DCs in clinical trials of cellular immunotherapy.

The present invention is further illustrated by the figures and examples:
**Figure 1**
   **Characteristics of immature Day 5 and Day 3 DCs.**
   **A.** Total number of immature Day 5 and Day 3 DCs that were generated from 75x10⁶ of PBMCs in CellGro. Data are presented as the mean ± SD for 7 independent experiments.
   **B**. Purity (percentage of CD11c⁺ DCs) and viability (percentage of DAPI⁻ cells) of immature and maturation stimuli-activated Day 5 and Day 3 DCs. are shown in FACS. One of seven performed experiments is presented.
   **C.** Morphology of Day 5 and Day 3 DCs. Day 5 DCs were larger in size and were more granular compared to Day 3 DCs.
**Figure 2**
   **The phenotype of Day 5 and Day 3 DCs activated for 24 h using LPS (control) or Poly(I:C).**
   **A.** The summary of six independent experiments ± SD and
   **B.** representative histograms (FACS analysis) are shown. Significant differences are shown (* P value = < 0.05, ** P value = < 0.01). Grey histograms represent the expression of maturation-associated molecules on immature DCs.
**Figure 3**
   **Cytokine production by mature Day 5 and Day 3 DCs.**
   Concentration of six cytokines - IL-12p70, IL-6, IL-1β, TNF-α, IL-10 and IFN-α was measured in supernatants from immature and mature Day 5 and Day 3 DCs. The data from seven independent experiments ± SD are shown. Significant differences are shown (* P value = < 0.05, ** P value = < 0.01).
**Figure 4**
   **Phagocytic ability of immature Day 5 and Day 3 DCs.**
   **A.** DiO-labeled immature Day 5 and Day 3 DCs were cocultured with DiD-labeled UVB-killed LNCap (prostate cancer) or OV90 (ovary cancer) cells at 37 °C for 24 h. As a control, DCs and tumor cells were cocultured on ice for 24 h (data not shown). DCs that phagocytosed killed tumor cells were identified as DiO⁺DiD⁺ double-positive cells. The results shown summarize five independent experiments ± SD. DiO and DID are two different kind of dialkylcarbocyanines. Dialkylcarbocyanines are cell membrane dyes that can be used to visualize dyed cells under specific light excitation. Specifically, in the present invention, DiD labels cells in green and DiO labels cells in red.
   **B.** Representative dot plots of phagocytosed killed LNCap and OV90 cells are shown.
   **C.** Fluorescent microscopy analysis of the phagocytosis. DiO-labeled immature Day 5 and Day 3 DCs were cocultured with Dil-labeled UVB-killed tumor cells at 37 °C for 24 h, and the engulfment of tumor cells was verified by fluorescent microscopy. DiO-labeled DCs are green and Dil-labeled tumor cells are red. Only phagocytosis of killed LNCap cells is shown. The almost complete lack of unphagocytosed killed tumor cells on the pictures is due to their weak adhesion to the slides; thus, they were washed out during the wash steps.
**Figure 5**
   **The induction of antigen-specific T cells by mature Day 5 and Day 3 DCs.**
   DCs were activated using maturation stimuli (LPS (control) or Poly(I:C)), pulsed with antigens and subsequently used as stimulators for the induction of antigen-specific T cells. The frequency of antigen-specific T cells was analyzed by intracellular IFN-γ staining. A, B. Induction of influenza MP- and CEF peptide mix-specific CD8⁺ T cells. The summary (A) and representative staining (B) of seven independent experiments are shown. The data are presented as the mean ± SD (A). Significant differences are shown (* P value = < 0.05, ** P value = < 0.01).
**Figure 6**
   **The induction of regulatory T cells from CD4⁺ CD25⁻ T cells by mature Day 5 and Day 3 DCs.**
   CD4⁺CD25⁻ T cells were obtained by depleting the CD25⁺ cells with CD25 beads (STEMCELL Technologies) (data not shown) and cultured alone or with activated peptide-pulsed Day 5 and Day 3 DCs. The frequency of CD4⁺CD25⁺FoxP3⁺ cells was analyzed from 7 day of cultures. CD25⁺FoxP3⁺ cells shown in graphs are expressed as the percentage of CD4⁺ cells. The results are expressed as percentage of CD25⁺FoxP3⁺ cells after 7 days of culture - percentage of CD25⁺FoxP3⁺ cells remaining after isolation. The summary (A) and representative staining (B) of three independent experiments are shown. The data are presented as the mean ± SD (A). Significant differences are shown (* P value = < 0.05, ** P value = < 0.01). (C) Columns represent proportions of Tregs demethylated in *FoxP3* TSDR.

### Example 1

### Generation of dendritic cells

Immature monocyte-derived DCs (moDCs) were generated as described above. Briefly, peripheral blood mononuclear cells (PBMCs) were isolated from buffy coats of healthy HLA-A2⁺ or HLA-A2⁻ donors by Ficoll-Paque PLUS gradient centrifugation (GE Healthcare) and monocytes were isolated by plastic adherence after 2 h of cell adhesion (75x10⁶ PBMCs) in Nunclon 75-cm² culture flasks (Nunc). To generate standard Day 5 moDCs, adherent monocytes were subsequently cultured for 5 days in serum-free CellGro DC media (CellGenix) in the presence of GM-CSF (Gentaur) at a concentration of 500 U/ml and 20 ng/ml of IL-4 (Gentaur). After 3 days of culture, fresh CellGro and cytokines were added to the culture flasks. After 5 days, immature DCs were seeded in Nunclon 48-well plates (5x10⁵ DCs in 500 µl of CellGro supplemented with cytokines per well) and activated for 24 h using Poly (I:C) (InvivoGen) at 25 µg/ml or LPS (Sigma-Aldrich) at 1 µg/ml. Alternatively, monocytes were cultured for 3 days in CellGro with GM-CSF (500 U/ml) and IL-4 (20 ng/ml), followed by incubation with the same maturation stimulus (Poly(I:C) or LPS) for 24 h (Day 3 DCs). Immature and mature DCs were used for further studies. For cocultures, a fraction of the PBMCs was cryopreserved, thawed and used for generation of DCs for restimulation. Non-adherent monocyte-depleted PBMCs were frozen and used as lymphocytes for cocultures with DCs.

### Example 2

### Flow cytometry

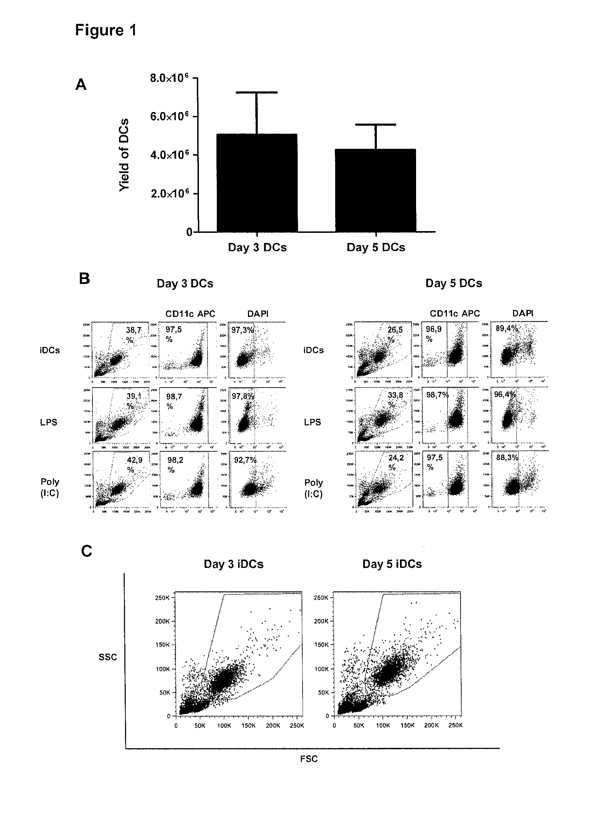

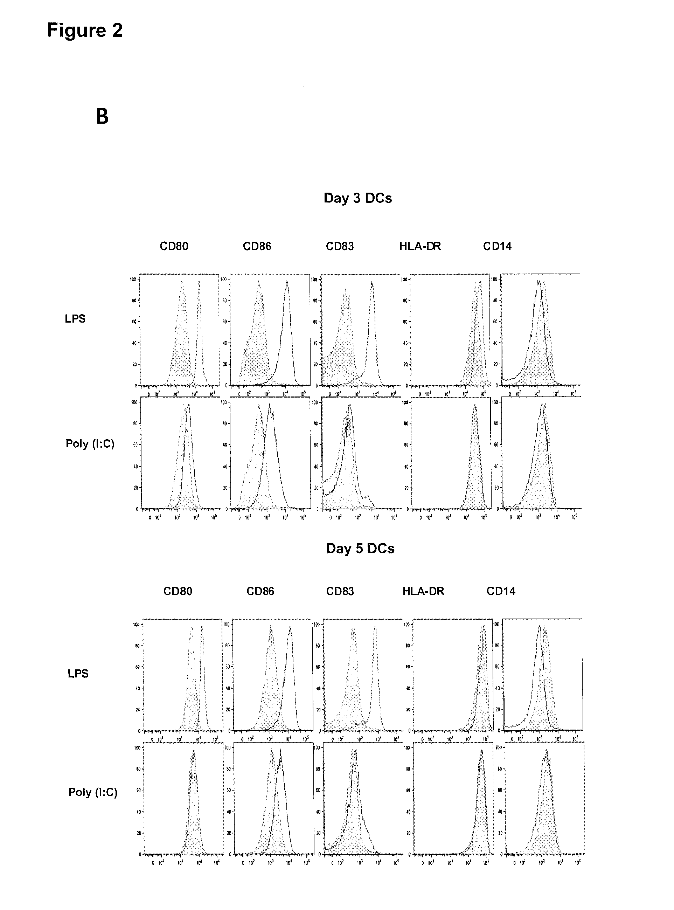

Immature and mature Day 5 and Day 3 DCs were phenotyped using the following monoclonal antibodies: CD80-FITC, CD86-PE, CD83-PE-Cy5 (Beckman Coulter), CD14-PE-Dy590, CD11c-APC (Exbio) and HLA-DR-PE-Cy7 (BD Biosciences). The cells were stained for 20 min at 4 °C, washed twice in PBS and analyzed using LSRFortessa (BD Biosciences) with FlowJo software (Tree Star). DCs were gated according to their FSC and SSC properties and as CD11c positive cells. Only viable DCs (DAPI negative cells) were included in the analysis. DAPI was purchased from Invitrogen. The results are shown in Fig. 1 B, Fig. 2 B.

### Example 3

### Cytokine measurements

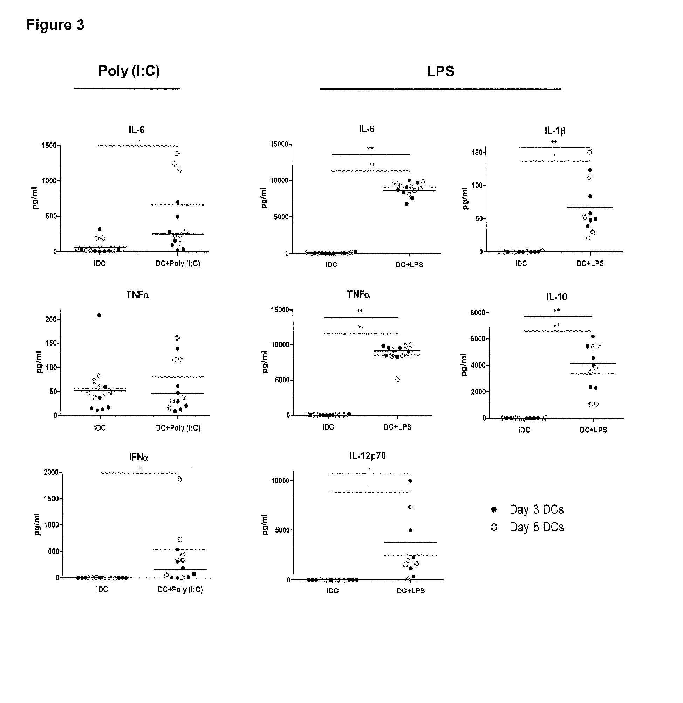

Cytokines in supernatants, released from immature and mature DCs, were measured using Milliplex Human Cytokine/Chemokine kit (Millipore) according to the manufacturer's instructions and analyzed by Luminex 200 (Luminex). Six cytokines were measured including IL-12p70, IL-6, IL-1β, TNF-α, IL-10 and IFN-α. Results are shown in Fig. 3.

### Example 4

### Uptake of UVB-irradiated cancer cells by DCs

LNCap prostate adenocarcinoma cell line and OV-90 ovarian cancer cell line were cultured in RPMI 1640 (Gibco) supplemented with 10% heat-inactivated FBS (PAA), 2 mM GlutaMAX I CTS (Gibco) and 100 U/ml penicillin + 100 µg/ml streptomycin (Gibco). For flow cytometry analysis of phagocytosis, tumor cells were harvested and labeled with Vybrant DiD cell labeling solution (Invitrogen). A fraction of the cells were labeled with Vybrant Dil cell labeling solution (Invitrogen) and used for fluorescent microscopy analysis. To prepare UVB-irradiated cells, stained LNCap or OV-90 cells were seeded in CellGro media in Nunclon 25-cm² culture flasks (Nunc) at a concentration of 4x10⁵ cells/ml and subjected to a 302 nm UVB-irradiation for 10 min to induce apoptosis. Cells were then incubated for 48 h at 37 °C with 5% CO₂ before use. To determine the uptake of UVB-irradiation killed tumor cells by DCs, immature Day 5 or Day 3 DCs were stained with Vybrant DiO cell labeling solution (Invitrogen) and cocultured with LNCap or OV-90 cells at a cell ratio of 5:1 in Nunclon U-bottom 96-well plates (Nunc) for 24 h at 37 °C with 5% CO₂. Parallel control cultures were set up for 24 h on ice to evaluate the passive transfer of dye or labeled tumor fragments to DCs. The phagocytic ability of DCs was evaluated by flow cytometry and fluorescent microscopy.

### Example 5

### Fluorescent microscopy

A suspension of labeled DCs and tumor cells was incubated on cover slips for 30 min at 37°C with 5% CO₂. Then, cells were washed in PBS, fixed with 4% paraformaldehyde for 25 min, washed twice in PBS and mounted on slides with ProLong Gold antifade reagent (Invitrogen).

### Example 6

### Expansion of antigen-specific T-lymphocytes and intracellular IFN-γ staining

Immature DCs were activated for 4 h with poly (I:C) (25 µg/ml) or LPS (1 µg/ml). Activated DCs were then pulsed with HLA-A2-restricted peptide from influenza MP (flu-MP) (GILGFVFTL) (Jpt peptide technologies) at 2,4 µg/ml or with CEF peptide mix (Jpt peptide technologies) at 2,5 µg/ml, DCs were incubated with peptides overnight. Non-adherent peripheral blood lymphocytes (PBL) (2 x 10⁵ in RPMI-1640 + 10% AB human serum (Invitrogen)) and the mature pulsed DCs (4 x 10⁴ in CellGro) were cocultured at a ratio of 5:1 in U-bottom 96-well plates for 7 days. A total of 20 U/ml of IL-2 (PeproTech) was added on days 3 and 5. On day 7, the lymphocytes were restimulated with fresh peptide-loaded DCs, and the frequency of antigen-specific T cells was determined using intracellular staining for IFN-γ. Brefeldin A (BioLegend) was added to block the extracellular release of IFN-γ 1,5 h after restimulation. After 3 h of incubation with Brefeldin A, the cells were washed in PBS, stained with anti-CD3-PerCP-Cy5.5 (eBioscience) and CD8-PE-Dy590 antibody (Exbio), fixed using Fixation Buffer (eBioscience), permeabilized with Permeabilization Buffer (eBioscience) and stained using anti-IFN-γ-FITC antibody (BD Biosciences). The cells were acquired using the LSRFortessa (BD Biosciences) and analyzed with FlowJo software (Tree Star).

### Example 7

### Isolation of naive CD4⁺ T cells and induction of Tregs by activated DCs

CD4⁺CD25⁻ T cells were isolated from non-adherent peripheral blood lymphocytes by depleting CD25⁺ cells using the EasySep Human CD4⁺CD25^{high} T cell isolation kit (STEMCELL Technologies). The purity of the cell population was verified by flow cytometry. Purified CD4⁺CD25⁻ T cells (2 x 10⁵ in RPMI-1640 + 10% AB human serum) and the mature peptide-pulsed DCs (4 x 10⁴ in CellGro) were cocultured at a ratio of 5:1 in U-bottom 96-well plates for 7 days. A total of 20 U/ml of IL-2 (PeproTech) was added on days 3 and 5. On day 7, the cells were stained with anti-CD4-PE-Cy7 (eBioscience) and anti-CD25-PerCP-Cy5.5 antibody (BioLegend), fixed with Fixation buffer (eBioscience), permeabilized with Permeabilization Buffer (eBioscience) and stained using anti-FoxP3-Alexa Fluor 488 antibody (eBioscience). The cells were acquired using LSRFortessa (BD Biosciences) and analyzed with FlowJo software (Tree Star).

### Example 8

### Extraction of genomic DNA and Quantitative real time PCR-based methylation assay

Genomic DNA (gDNA) was isolated from a lysate of 2 x 10⁶ autologous induced Tregs using the PureLink Genomic DNA Mini kit (Invitrogen, Carlsbad, USA). Concentrations of extracted gDNA were measured with the Nanodrop^{©} 2000c UV-Vis spectrophotometer (Thermo Scientific, Waltham, USA). One to 2 micrograms of gDNA was treated with sodium bisulfite using the MethylCode Bisulfite Conversion kit (Invitrogen, Carlsbad, USA). The quantitative real-time PCR amplification assay of methylated and demethylated *FoxP3* TSDR sequences had a final volume of 10 µl and contained 2 µl of bisulfite treated gDNA, 0,04 µl (∼ 0,2 U) of Platinum Taq DNA polymerase (Invitrogen, Carlsbad, USA), 1 x concentrated PCR buffer without MgCl₂, 3 mM MgCl₂, 0,2 mM dNTP (each), 1 µM primers and 0,2 µM TaqMan^{©} probe. PCR reactions were performed using a CFX 96 cycler (BioRad, Hercules, USA) with an appropriate cycling protocol (95 °C for 3 minutes followed with 50 cycles at 95 °C for 15 seconds and 60 °C for 1 minute). Methylation-specific and demethylation-specific primers and TaqMan© probes were commercially synthesized (TIB MOLBIOL, Berlin, Germany). Amounts of methylated and demethylated *FoxP3* TSDR DNA were estimated from calibration curves by crossing points linear regression using the

second derivative maximum method. The proportion of cells with a demethylated *FoxP3* TSDR was calculated as the ratio of demethylated *FoxP3* TSDR DNA to the sum of methylated and demethylated *FoxP3* TSDR DNA.

### Example 9

### Plasmid standards

DNA fragments of methylated and demethylated *FoxP3* TSDR (86 bp) were cloned into the plasmid pUC18 (Amersham Biosciences, Amersham, UK) using *Hind*III and *Eco*RI restriction sites. The identity of the plasmid constructs was verified by sequencing followed with plasmid DNA purification using Wizard Plus Midipreps kit (Promega, Madison, USA) and dilution to seven final concentrations of 100, 10, 1 pg/µl, 100, 10, 1 and 0,1 fg/µl, representing a range of 3,34 x 10⁻⁷ to 3,34 x 10⁻¹ plasmid copies. Serial dilutions demonstrated the specificity of the assay and were used as standards for the quantification of methylated and demethylated *FoxP3* TSDR.

### Statistical analysis

The data were analyzed by One-Way ANOVA with Dunnett's multiple comparison post hoc test and Student's unpaired two-tailed t test using GraphPad Prism 5. The results were considered statistically significant when p < 0.05.

### Example 10

### The yield, viability and morphology of immature Day 5 and Day 3 DCs

The characteristics of Day 5 and Day 3 DCs differentiated in CellGro from 75x10⁶ PBMCs were compared. There were no significant differences detected either in the yield of immature Day 5 and Day 3 DCs (Figure 1A) or their viability (Figure 1B). In contrast, FSC/SSC analysis showed that Day 5 DCs were larger in size and more granular than Day 3 DCs (Figure 1C).

### Example 11

### Immunophenotype of Day 5 and Day 3 DCs following maturation

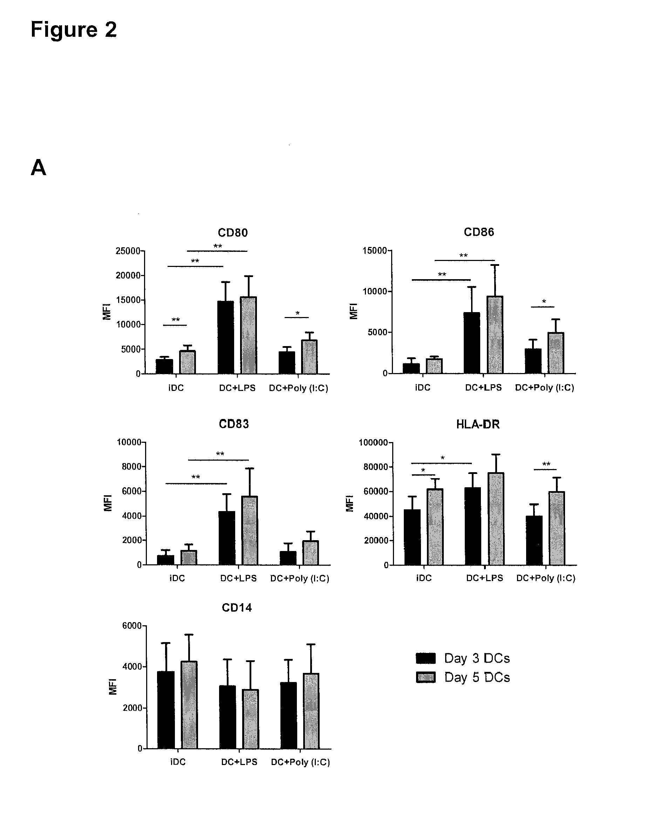

To determine the capacity of immature Day 5 and Day 3 DCs to be activated by Toll-like receptor ligands (Poly(I:C) or LPS), the expression of costimulatory molecules CD80 and CD86, CD83 and MHC class II was evaluated. As a control, immature DCs were incubated for 24 h in the presence of IL-4 and GM-CSF. The phenotype analyses revealed that exposure of immature DCs to LPS caused the significantly upregulated the expression of all costimulatory molecules and HLA-DR (Figure 2); additionally, the downregulation of CD14 expression was not statistically significant. No significant differences between LPS-activated Day 5 and Day 3 DCs in the expression of maturation-associated molecules were detected. Although, Poly (I:C) induced weaker phenotypic maturation compared to LPS, and Poly(I:C)-activated Day 5 DCs expressed higher levels of CD80, CD86 and HLA-DR compared to Day 3 DCs.

### Example 12

### Cytokine production by mature Day 5 and Day 3 DCs

The production of IL-12p70, IL-6, IL-1β, TNF-α, IFN-α and IL-10 by Poly(I:C)- or LPS-activated Day 5 and Day 3 DCs was measured. As a control, immature DCs were incubated for 24 h in the presence of IL-4 and GM-CSF. Cytokine production was stronger when LPS was used as a maturation stimulus (Figure 3). DCs activated by LPS produced high levels of all cytokines, except for IFN-α, when compared to immature and Poly (I:C)-activated DCs (as there was a high variability in IL-12p70 secretion between individual donors, difference in IL-12p70 secretion was not statistically significant), and there were no significant differences between Day 5 and Day 3 DCs. In contrast, Poly(I:C) only induced production of IL-6 and IFN-α. TNF-α was only weakly secreted by Poly(I:C)-activated DCs and at the same levels as by immature DCs. The levels of IL-1β and IL-10 were almost undetectable. Furthermore, there was an absence of IL-12p70 in culture supernatants from Poly(I:C)-activated DCs. When we compared Day 5 and Day 3 Poly(I:C)-activated DCs, Day 5 DCs secreted relatively high amounts of IL-6 and IFN-α; however, this difference was not statistically significant.

### Example 13

### Phagocytic ability of immature Day 5 and Day 3 DCs

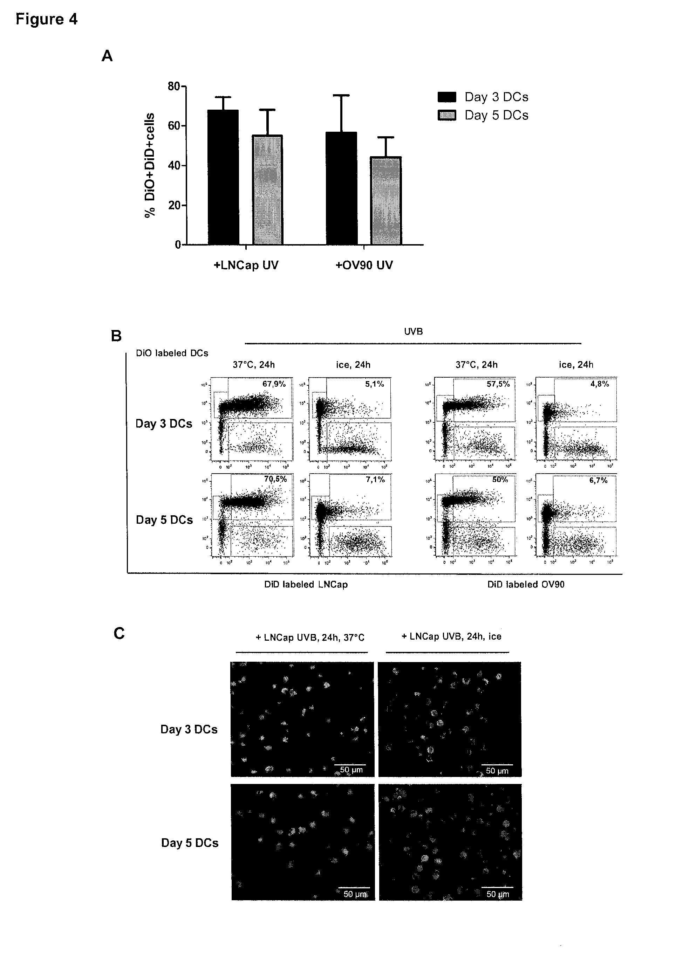

To assess the uptake of killed tumor cells via phagocytosis, immature DiO labeled Day 5 and Day 3 DCs were cultured in the presence of IL-4 and GM-CSF and incubated with DiD or Dil labeled UVB-irradiation killed LNCap or OV-90 cells for 24 h at 37 °C and 5% CO₂. As a control, DCs and tumor cells were incubated for 24 h on ice. DCs that phagocytosed killed tumor cells were identified as DiO⁺DiD⁺ double-positive cells. No significant differences in the uptake of UVB-irradiation killed LNCap and OV-90 cells (Figure 4) were detected, and immature Day 5 and Day 3 DCs showed similar phagocytic capabilities. The uptake of killed tumor cells by immature DCs was confirmed to be an active process, as the passive transfer of tumor fragments onto DC surfaces on ice was minimal (< 15%, data not shown). Furthermore, fluorescent microscopy revealed that killed LNCap and OV-90 cells, or their fragments, were present inside the DCs, indicating that immature Day 5 and Day 3 DCs were able to fully engulf killed tumor cells (Figure 4C).

### Example 14

### In vitro capacity of mature Day 5 and Day 3 DCs to induce antigen-specific T cells

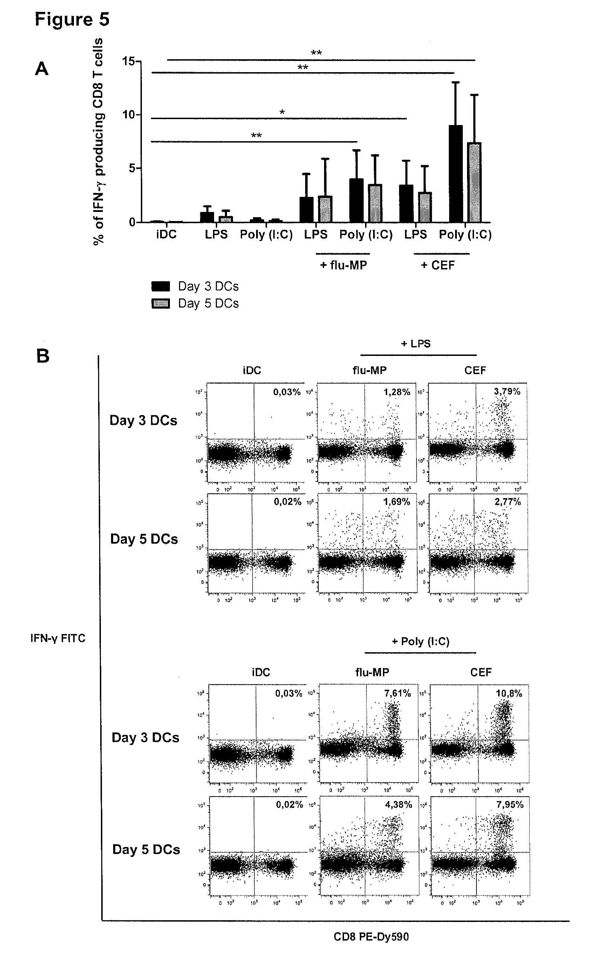

The capacity of mature DCs to expand influenza MP- and CEF peptide mix-specific T cells was evaluated. Mature Day 5 and Day 3 DCs generated in CellGro media were pulsed with antigens and used for the expansion of antigen-specific T cells for 7 days. The frequency of IFN-γ-producing T cells was analyzed one week later, after restimulation with identical peptide-loaded DCs. As a control, lymphocytes were cocultured with immature DCs or Poly(I:C)- or LPS-activated DCs without added peptides. Antigen-loaded Poly(I:C), as well as LPS-activated, Day 3 DCs were equally effective at inducing the expansion of antigen-specific T cells compared to Day 5 DCs (Figure 5). The strongest response was observed when CEF peptide mix-loaded Poly(I:C)-activated DCs were used for the stimulation of autologous T cells. The results showed that Day 3 DCs were as potent as Day 5 DCs in stimulating antigen-specific T cells. Furthermore, Poly(I:C)-activated DCs were slightly more efficient at expanding antigen-specific T cells compared to LPS-activated DCs.

### Example 15

### Capacity of mature Day 5 and Day 3 DCs to induce regulatory T cells from CD4⁺CD25⁻ T cells

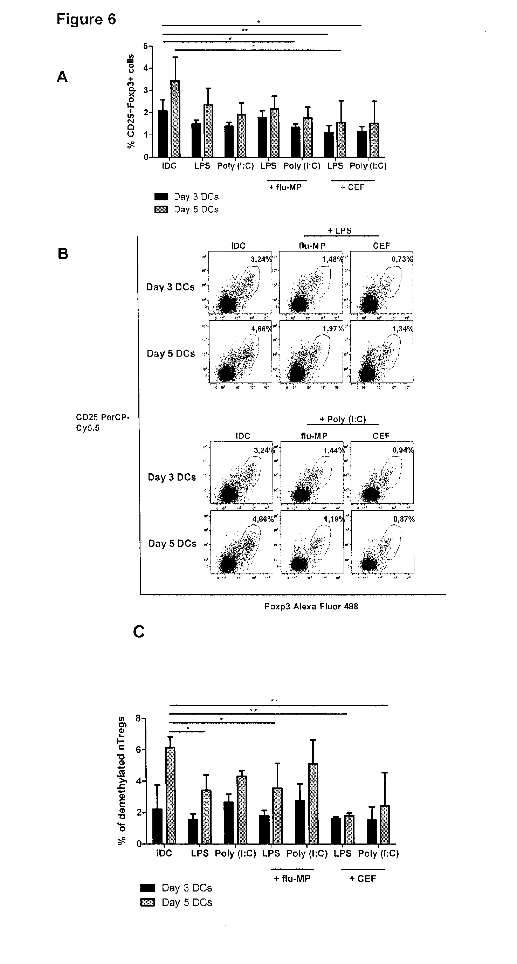

The capacity of activated peptide-pulsed Day 5 and Day 3 DCs to induce regulatory T cells (Tregs) was tested. Tregs induced after 7 days of DC and T cell cocultures were determined to be CD4⁺CD25^{high}FoxP3⁺ T cells. In general, immature DCs generated the highest numbers of Tregs. There was not a significant difference in the numbers of Tregs induced by DCs that were activated by either LPS or Poly(I:C). LPS or Poly(I:C)-activated Day 3 DCs generated slightly lower numbers of Tregs compared to LPS or Poly(I:C)-activated Day 5 DCs (Figure 6A, B). This finding was further confirmed using a quantitative real-time PCR-based methylation assay, which assessed the percentage of stable Tregs that were demethylated at *FoxP3* TSDR (Figure 6C).

## Claims

1. In vitro method for preparing dendritic cells as antigen-presenting cells for use in immunotherapy whereby the method comprises the following steps:
a) isolation of peripheral blood mononuclear cells,
b) isolation of monocytes from peripheral blood mononuclear cells by adherence to plastic material,
c) culturing the monocytes in serum free medium for the generation of dendritic cells for up to 80 hours and
d) activation of the cultured monocyte cells / dendritic cells obtained from step c) with 10-50 µg/ml Poly(I:C) for up to 28 hours.

2. In vitro method according to claim 1 wherein the monocytes are cultured in serum-free medium for the generation of dendritic cells for 65 to 80 hours.

3. In vitro method according to claim 1 or 2 wherein the monocytes cultured in serum-freem medium for the generation of dendritic cells according to step c) are activated in step d) with 10-50 µg/ml Poly(I:C) for 4-28 hours.

4. In vitro method according to any of claims 1 to 3 wherein monocytes are cultivated in serum-free medium and wherein the dendritic cells are activated in serum-free medium.

5. In vitro method for preparing dendritic cells according to claims 1 to 4 wherein the peripheral blood mononuclear cells are isolated from the buffy coat fraction of centrifuged blood of a donor by gradient centrifugation.

6. In vitro method according to claim 1 to 5 wherein the monocytes are isolated by adhesion of the cells to culture flasks made from polystyrene.

7. In vitro method according to any of claims 1 to 6, **characterized in that** the serum free medium for dendritic cells contains salts, sugars, amino acids, vitamins, buffers, human plasma derived transferin, human plasma derived albumin, human recombinantly produced insulin whereby said medium has an osmolality of 260-320 mOsm/kg H₂O, and a pH value of 7.2 to 7.5.

8. In vitro method according to any of claims 1 to 7, **characterized in that** the serum free medium for the cultivation of dendritic cells contains GM-CSF in an amount of 350 to 700 units/ml and 10-50 ng/ml of IL-4.

9. In vitro method according to any of claims 1 to 8, **characterized in that** the monocytes are cultured in serum free medium for dendritic cells for 70 to 74 hours.

10. In vitro method according to any of claims 1 to 9, **characterized in that** the activation of the dendritic cells is performed for 22-26 hours.

11. Dendritic cells prepared according to an in vitro method according to any of claims 1 to 10 for use in tumor vaccination.

12. Dendritic cells according to claim 11 for use for the vaccination of a tumor selected from the group consisting of prostate cancer, breast cancer and lung cancer.
